# EUROPÄISCHE PATENTSCHRIFT

(11) **EP 2 757 942 B1**
(45) Veröffentlichungstag und Bekanntmachung des Hinweises auf die Patenterteilung: **04.03.2020**
(21) Anmeldenummer: 12778606.9
(22) Anmeldetag: 21.09.2012
(51) Int. Cl.: A61B 5/053, A61B 5/021, A61B 5/0408

(54) **DIAGNOSTISCHE MESSVORRICHTUNG**
DIAGNOSTIC MEASUREMENT DEVICE
DISPOSITIF DE MESURE DIAGNOSTIQUE

(30) Priorität: 21.09.2011 DE 102011113841
(43) Veröffentlichungstag der Anmeldung: 30.07.2014
(73) Patentinhaber: Flore, Ingo, 44141 Dortmund (DE)
(72) Erfinder: KIM, Yoon, Ok, 58239 Schwerte (DE); CHO, Ok, Kyung, 58239 Schwerte (DE)
(74) Vertreter: Isfort, Olaf
(86) Internationale Anmeldenummer: PCT/EP2012/003957
(87) Internationale Veröffentlichungsnummer: WO 2013/041232

(56) Entgegenhaltungen:
- WO-A1-2008/061788
- WO-A1-2011/022068
- WO-A2-2010/128500
- DE-A1- 19 715 421
- US-A- 5 467 768
- US-A1- 2004 181 141
- US-A1- 2007 255 122

## Beschreibung

Die Erfindung betrifft eine Messvorrichtung zur nicht-invasiven Bestimmung von wenigstens einem physiologischen Parameter, mit einer diagnostischen Sensoreinheit zur Erzeugung von Messsignalen, und mit einer Auswertungseinheit zur Verarbeitung der Messsignale gemäß dem Oberbegriff des Patentanspruchs 1. Außerdem betrifft die Erfindung ein Verfahren zur nicht-invasiven Bestimmung von wenigstens einem physiologischen Parameter.

Eine derartige Messvorrichtung ist aus der WO 2011/022068 A1 bekannt.

Eine weitere ähnliche Messvorrichtung ist beispielsweise aus der WO 2008/061788 A1 bekannt. Die vorbekannte Messvorrichtung weist eine Sensoreinheit auf, die in die Tastatur eines Computers oder in ein mobiles Gerät der Unterhaltungs- oder Kommunikationstechnik integriert ist. Dabei umfasst die diagnostische Sensoreinheit verschiedene Messmodalitäten, nämlich eine optische Messeinheit, eine EKG-Einheit, einen Temperatursensor und/oder eine bioelektrische Impedanzeinheit. Die Kombination der verschiedenen Messmodalitäten ermöglicht eine kombinierte Auswertung der entsprechenden Messsignale mittels einer in geeigneter Weise programmierten Auswertungseinheit. Die Kombination gewährleistet dabei eine hohe Effizienz und Zuverlässigkeit bei der Erkennung von pathologischen Störungen. Insbesondere ermöglicht die vorbekannte Messvorrichtung eine nicht-invasive indirekte Messung von metabolischen Parametern. Letztlich ist damit die nicht-invasive Messung der Glukosekonzentration bzw. des Blutglukosespiegels möglich.

Aufgabe der Erfindung ist es, eine Messvorrichtung mit gegenüber dem Stand der Technik erweiterter Funktionalität bereit zu stellen. Aufgabe der Erfindung ist es weiterhin, eine Messvorrichtung mit möglichst einfach aufgebauter diagnostischer Sensoreinheit bereit zu stellen, so dass die Messvorrichtung insgesamt kostengünstig hergestellt werden kann.

Zur Lösung mindestens einer der genannten Aufgaben schlägt die Erfindung ausgehend von einer Messvorrichtung der eingangs genannten Art vor, dass die Messelektrode an der diagnostischen Sensoreinheit beweglich gelagert ist.

Auf diese Weise ergibt sich eine besonders elegante Integration des Drucksensors.

Der bei der erfindungsgemäßen Messvorrichtung vorgesehene Drucksensor detektiert einen uniaxialen Druck. Der Drucksensor detektiert, anders ausgedrückt, eine von dem Körpergewebe auf den Drucksensor ausgeübte Kraft in einer bestimmten Richtung, meist in der Richtung senkrecht zur Körperoberfläche, an der der Drucksensor zur Anlage kommt. Unter einem Drucksensor im Sinne der Erfindung ist somit auch ein Kraftsensor zu verstehen. Gemäß der Erfindung wird der von dem zu untersuchenden Körpergewebe lokal auf den Drucksensor ausgeübte Druck detektiert. Dabei wird davon ausgegangen, dass der Drucksensor eine (vergleichsweise kleine) Anlagefläche nutzt, die an der Körperoberfläche anliegt und über die der vom Körpergewebe ausgeübte Druck detektiert wird. Die Größe der Anlagefläche kann zum Beispiel im Bereich von 1 mm² bis 10 cm² liegen. Der lokal auf dem Drucksensor ausgeübte Druck im Sinne der Erfindung ist der über diese Anlagefläche des Drucksensors detektierte Druck.

Kern der Erfindung ist es , den Drucksensor als diagnostischen Sensor zu verwenden. Beispielsweise kann die Auswertungseinheit der erfindungsgemäßen Messvorrichtung eingerichtet sein, aus dem zeitlichen Verlauf des Messsignals des Drucksensors wenigstens einen der folgenden physiologischen Parameter abzuleiten: Druckvolumenpuls, Pulsamplitude, Pulsbreite, Pulsdauer, Durchblutung, Blutdruck, Pulsdruck, Herzfrequenz, Pulswellengeschwindigkeit, Körpertemperatur, metabolisch erzeugte Wärme. Der Verlauf des Messsignals des Drucksensors liefert unmittelbar den Druckvolumenpuls. Daraus können dann diverse weitere physiologische Parameter bestimmt werden. Beispielsweise korreliert die Pulsamplitude mit der Körpertemperatur, so dass, vorzugsweise nach entsprechender Kalibrierung, aus dem Messsignal des Drucksensors die Körpertemperatur abgeleitet werden kann, nämlich die Körperoberflächentemperatur und/oder die Körperdurchschnittstemperatur, die arterielle Bluttemperatur, die Körperkerntemperatur oder die metabolisch produzierte Wärmemenge. Weiterhin können Rückschlüsse gezogen werden auf die vom Körperinneren zum Ende der arteriellen Kapillaren fließende Wärmemenge sowie auf die Temperatur im Bereich der arteriell-venösen Kapillaren bzw. des diese umgebenden Körpergewebes.

Zur Gewinnung des Messsignals mittels der erfindungsgemäßen Messvorrichtung muss der wenigstens eine Drucksensor direkt oder indirekt mit der Körperoberfläche des Benutzers in Kontakt gebracht werden, beispielsweise in dem der Benutzer einen Finger auf die mit dem Drucksensor ausgestattete Vorrichtung auflegt. Ebenso ist es denkbar, dass der Drucksensor mittels geeigneter Mittel an die Oberfläche des Körpergewebes angepresst wird. Hierzu kann ein Halteclip an sich bekannter Art oder eine elastische Manschette dienen. Ebenso denkbar ist es, dass, zum Beispiel durch medizinisch geschultes Personal, die Messvorrichtung mit dem Drucksensor am Körper des Benutzers entlang geführt wird, um an verschiedenen Stellen am Körper den Druck zu messen. Nach Anlegen des Drucksensors am Körper des Benutzers wird das Messsignal während eines vorgegebenen Zeitraums (mehrere Sekunden bis mehrere Minuten, oder auch kontinuierlich) erfasst. Der Zeitverlauf des Messsignals wird dann, wie oben beschrieben, ausgewertet.

Bei einer bevorzugten Ausgestaltung weist die Sensoreinheit der erfindungsgemäßen Messvorrichtung eine optische Messeinheit auf, die wenigstens eine Strahlungsquelle zur Bestrahlung des Körpergewebes und wenigstens einen Strahlungssensor zur Detektion der von dem Körpergewebe gestreuten und/oder transmittierten Strahlung umfasst, wobei die Auswertungseinheit zur Ableitung des wenigstens einen physiologischen Parameters zumindest aus dem Messsignal des Drucksensors und der optischen Messeinheit eingerichtet ist. Die optische Messeinheit kann wie in der oben zitierten WO 2008/061788 A1 beschrieben ausgestaltet sein. Mittels der optischen Messeinheit kann zum Beispiel die arterielle Sauerstoffsättigung bestimmt werden. Der mittels des Drucksensors bestimmte Druckvolumenpuls kann dabei die optische Messung ergänzen oder teilweise ersetzen, da der Druckvolumenpuls unabhängig von der Lichtabsorption einzelner Körpergewebe- oder Blutbestandteile bestimmt werden kann. Somit kann das Messsignal des Drucksensors als Referenzwert dienen und dadurch die Messgenauigkeit erhöhen. Außerdem ermöglicht die Erfassung des Messsignals des Drucksensors die Vereinfachung der optischen Messung. Beispielsweise kann die Messung auf eine geringere Anzahl unterschiedlicher Wellenlängen reduziert werden.

Gemäß einer weiteren bevorzugten Ausgestaltung umfasst die Sensoreinheit der Messvorrichtung einen Temperatursensor, wobei die Auswertungseinheit zur Ableitung des wenigstens einen physiologischen Parameters zumindest aus den Messsignalen des Drucksensors und des Temperatursensors eingerichtet ist. Aus der zitierten WO 2008/061788 A1 ist es bekannt, dass aus kombinierter Temperatur- und optischer Messung die Glukosekonzentration im Blut ermittelt werden kann. Entsprechend kann die Temperatur- bzw. Wärmemessung die weiteren Messmodalitäten sinnvoll ergänzen. Die Messsignale des Temperatursensors ermöglichen Rückschlüsse auf den lokalen Wärmeaustausch und damit auf die lokale Stoffwechselaktivität. Außerdem ist der Temperatursensor zur Bestimmung der lokalen Durchblutung geeignet.

Bei einer weiteren bevorzugten Ausgestaltung weist die Sensoreinheit der erfindungsgemäßen Messvorrichtung eine EKG-Einheit zur Erfassung eines EKG-Signals über zwei oder mehr EKG-Elektroden auf, wobei die Auswertungseinheit zur Ableitung des wenigstens einen physiologischen Parameters zumindest aus den Messsignalen des Drucksensors und der EKG-Einheit eingerichtet ist. Durch die EKG-Einheit wird der Funktionsumfang der erfindungsgemäßen Messvorrichtung erweitert. Die Auswertungseinheit der Messvorrichtung kann zum Beispiel zur Auswertung des zeitlichen Verlaufs des Druckvolumenpulses und des EKG-Signals eingerichtet sein. Daraus kann wiederum die Pulswellengeschwindigkeit ermittelt werden, was unter anderem Rückschlüsse auf den Blutdruck ermöglicht. Die Kombination aus Drucksensor und EKG-Einheit ermöglicht, anders ausgedrückt, eine automatisierte funktionale Bewertung des Zustands des Gefäßsystems des Benutzers der Messvorrichtung.

Die Sensoreinheit der erfindungsgemäßen Messvorrichtung weist eine bioelektrische Impedanzmesseinheit auf, wobei die Auswertungseinheit zur Ableitung des wenigstens einen physiologischen Parameters zumindest aus dem Messsignal des Drucksensors und der bioelektrischen Impedanzmesseinheit eingerichtet ist. Insbesondere kann die bioelektrische Impedanzmesseinheit zur lokalen Bioimpedanzmessung ausgebildet sein. Die Kombination aus Drucksensor und bioelektrischer Impedanzmesseinheit ermöglicht die Bestimmung der im Körpergewebe enthaltenen Wassermenge, des Anteils der fettfreien Masse am Körpergewebe sowie des Körperfettanteils, ohne dass notwendigerweise die Gesamtkörpermasse bestimmt bzw. eingegeben werden muss. Die bioelektrische Impedanzmessung ermöglicht weiterhin, wie in der zitierten WO 2008/061788 A1 im Detail beschrieben, ggfs. in Kombination mit weiteren Messmodalitäten, eine nicht-invasive Bestimmung der Glukosekonzentration.

Erfindungsgemäß ist der Drucksensor mechanisch an eine Messelektrode der bioelektrischen Impedanzmesseinheit gekoppelt und kann so den von dem zu untersuchenden Körpergewebe über die Messelektrode lokal auf den Drucksensor ausgeübten Druck detektieren. Auf diese Weise lässt sich der Drucksensor besonders elegant integrieren. Die Messelektrode und der Drucksensor benutzen für die Messsignalerfassung ein- und dieselbe Anlagefläche am Körper. Zur Ermöglichung der Druck- bzw. Kraftmessung sollte die Messelektrode dabei an der diagnostischen Sensoreinheit beweglich (z. B. federnd) gelagert sein. Der eigentliche Drucksensor kann dann zum Beispiel ein piezoresistives Element umfassen. Das piezoresistive Element kann vorteilhaft unter der Messelektrode der bioelektrischen Impedanzeinheit angeordnet sein. Auf diese Weise wird die auf die Elektrode ausgeübte Kraft bzw. der ausgeübte Druck direkt in ein elektrisches Messsignal umgewandelt.

Alternativ oder zusätzlich kann der Drucksensor an eine Messelektrode der EKG-Einheit gekoppelt sein.

Die erfindungsgemäße Messvorrichtung bietet eine Vielzahl von Vorteilen für die Bestimmung eines oder mehrerer physiologischer Parameter. Die Messgenauigkeit kann erhöht werden. Der Drucksensor kann als Referenz verwendet werden, um die Fehler der Messsignale, die mittels anderer Messmodalitäten gewonnen werden, aufzudecken. Der Drucksensor hat weiterhin den Vorteil, dass dieser mit einfachen elektronischen Schaltungen nutzbar gemacht werden kann. Außerdem sind Drucksensoren energiesparend und sowohl produktions- als auch anwendungstechnisch einfach zu handhaben. Drucksensoren können in kleiner Größe realisiert werden und haben eine niedrige Fehlertoleranz. Der Einsatz eines oder mehrerer Drucksensoren bei der erfindungsgemäßen Messvorrichtung als einzige Messmodalität ist genauso möglich wie der Einsatz zur Ergänzung anderer Messmodalitäten. So können zum Beispiel der Druckvolumenpuls, die Pulsfrequenz und weitere damit zusammenhängende Parameter schon allein und ausschließlich aus dem Messsignal des Drucksensors bestimmt werden, was für viele praktische Anwendungen ausreicht. Entsprechend kann für eine Vielzahl von Anwendungszwecken auf eine (aufwendigere) optische Messung oder eine EKG-Messung verzichtet werden.

Bei einer Messvorrichtung mit diagnostischer Sensoreinheit, die wie oben beschrieben ausgebildet ist, ist die Auswertungseinheit zur Ableitung des wenigstens einen physiologischen Parameters als Funktion der Tiefe im Körpergewebe eingerichtet Oberflächenanalytische und körperdurchschnittsanalytische Verfahren zur Bestimmung von physiologischen Parametern sind allgemein bekannt und üblich. Von besonderem Vorteil ist demgegenüber die Tiefenanlyse bzw. die Tiefenprofilanalyse oder die Tiefenquerschnittsprofilanlyse, bei denen ein oder mehrere physiologische Parameter ortsaufgelöst, insbesondere tiefenaufgelöst ermittelt werden. Besonders bevorzugt erfolgt eine tiefenaufgelöste und zeitabhängige Erfassung von physiologischen Parametern. Die so gewonnenen Daten ermöglichen es detailliert, im Körpergewebe ablaufende physiologische, insbesondere metabolische Prozesse quantitativ und ortsaufgelöst zu analysieren. Dies wiederum ermöglicht ein genaueres, für den Patienten bzw. für den Benutzer der Messvorrichtung bequemeres sowie kosten- und aufwandseffizienteres Vorgehen bei der Diagnose. Im Sinne der Erfindung wird ein nicht-invasives Tiefenanalyseverfahren zur Bestimmung von physiologischen Parametern bereitgestellt, bei dem nicht-invasiv physiologische Parameter und entsprechende biochemische Prozesse im Inneren des menschlichen Körpers tiefenabhängig bestimmt werden. Insbesondere ermöglicht eine Tiefenprofilanalyse durch Anwendung der oben beschriebenen Messmodalitäten (Druck, Temperatur, optische Messung, bioelektrische Impedanz, EKG), jeweils einzeln oder in Kombination, die Ermittlung relevanter physiologischer Parameter zur Bestimmung der Zusammensetzung des Körpergewebes, beispielsweise des die Kapillaren umgebenden Gewebes. Das Wissen um die Zusammensetzung des Körpergewebes kann wiederum als Parameter in die Berechnung bei der Ableitung von physiologischen Parametern aus den Messsignalen herangezogen werden. Eine spezielle Variante der Tiefenprofilanalyse ist die Tiefenquerschnittsprofilanalyse. Bei dieser wird der Querschnitt des Körpergewebes in Richtung der Tiefe, d. h. senkrecht zur Körperoberfläche, von einem Startpunkt aus analysiert, wobei dieser Startpunkt nicht notwendigerweise direkt an der Körperoberfläche liegen muss. Beispielsweise kann eine Tiefenquerschnittprofilanalyse durch bioelektrische Impedanzmessung erfolgen, wobei der Abstand bzw. die relative Position der verwendeten Elektroden zum untersuchten Körpergewebe, die Stärke des zur Impedanzmessung aufgeprägten Stroms und/oder die Messfrequenz variiert werden. Für die Tiefenquerschnittsprofilanalyse eignet sich somit eine erfindungsgemäße Messvorrichtung, die die oben aufgeführten Messmodalitäten einzeln oder in Kombination umfasst, besonders gut. Einzelne oder mehrere der folgenden physiologischen Parameter können gemäß der Erfindung tiefenanalytisch, tiefenprofilanalytisch oder tiefenquerschnittsprofilanalytisch erfasst werden: Durchblutung, Blutdruck, Pulsamplitude, Pulsdruck, Pulsbreite, Blutmenge, Körperoberflächentemperatur, Körperdurchschnittstempteratur, arterielle Bluttemperatur, Körperkerntemperatur, vom Körperinneren zu den Kapillarenden transportierte Wärmemenge, Temperatur im Bereich der arteriell-venösen Kapillaren bzw. des diese umgebenden Gewebes, Pulswellengeschwindigkeit, vom Metabolismus produzierte Wärmemenge, arterielle Sauerstoffsättigung, Sauerstoffsättigung im Gewebe, Sauerstoffverbrauch, Körperwassermasse, Anteil der fettfreien Masse am Körpergewebe, Körperfettanteil, Glukosekonzentration usw..

Dabei kann, was ein wesentlicher Vorteil der Erfindung gegenüber dem Stand der Technik ist, die für die Messvorrichtung benötigte Hardware sehr kompakt ausgeführt werden. Die einzelnen Sensoren benötigen einen Bauraum, der zum Beispiel kleiner als 2 cm x 2 cm x 2 cm sein kann. Einige Sensoren sind sogar mit einem Bauvolumen von weniger als 1 cm x 1 cm x 1 cm oder sogar noch kleiner zu realisieren. Selbst eine Kombination aus mehreren Messmodalitäten in der erfindungsgemäßen Messvorrichtung kann als kompaktes, portables Gerät ("handheld") zur Tiefenanalyse oder zur Tiefenprofilanalyse realisiert werden. Die Kantenlänge des Gerätes kann zum Beispiel weniger als 10 bis 20 cm betragen. Sogar kleinere Abmessungen sind denkbar. Die Hardwarekosten und damit die Diagnosekosten sind bei Verwendung der erfindungsgemäßen Messvorrichtung deutlich geringer als bei Anwendungen herkömmlicher ortsaufgelöster Diagnoseverfahren (z. B. Computertomographie).

Die oben erwähnte zu Grunde liegende Aufgabe wird auch durch ein Verfahren zur nicht-invasiven Bestimmung von wenigstens einem physiologischen Parameter gelöst, bei dem der von einem zu untersuchenden Körpergewebe lokal auf einem Drucksensor ausgeübte Druck detektiert wird, wobei der wenigstens eine physiologische Parameter aus dem detektierten Druck abgeleitet wird.

Ausführungsbeispiele der Erfindung werden im Folgenden anhand der Zeichnungen näher erläutert. Es zeigen:
- Figur 1: Messsignal des Drucksensors der erfindungsgemäßen Messvorrichtung als Funktion der Zeit;
- Figur 2: Ausführungsbeispiel einer Sensoreinheit der erfindungsgemäßen Messvorrichtung mit Messelektrode und Drucksensor;
- Figur 3: weiteres Ausführungsbeispiel einer Sensoreinheit der erfindungsgemäßen Messvorrichtung mit matrixförmiger Anordnung von Messelektroden;
- Figur 4: weitere Ausführungsbeispiele mit unterschiedlichen Konfigurationen der Messelektroden.

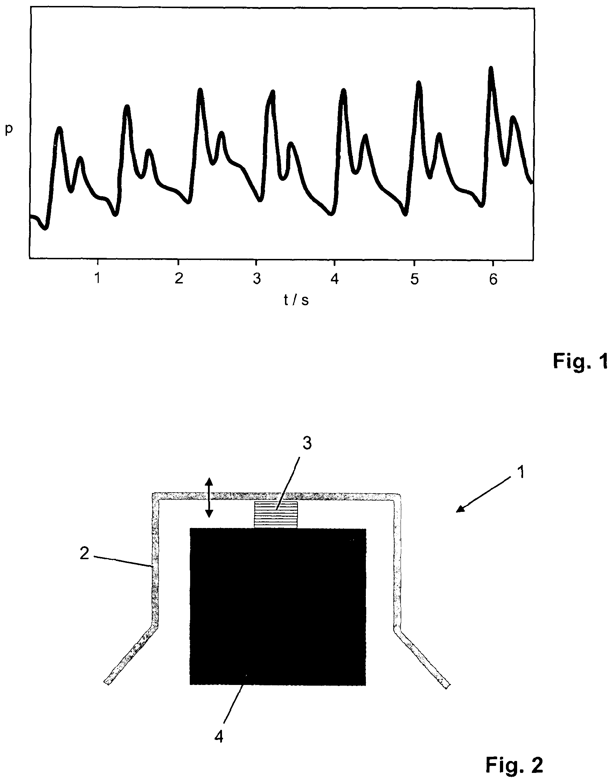

Die Figur 1 illustriert die Ableitung des Druckvolumenpulses aus dem Messsignal eines Druck- bzw. Kraftsensors, der erfindungsgemäß Bestandteil einer diagnostischen Sensoreinheit ist. Der Drucksensor liegt an der Körperoberfläche eines Patienten in dem zu untersuchenden Bereich an, so dass der Drucksensor den von dem Körpergewebe lokal auf den Drucksensor ausgeübten Druck detektiert, und zwar, wie in Figur 1 zu sehen ist, als Funktion der Zeit t. Das Messsignal ist der Druck p. Der Zeitverlauf des Messsignals p entspricht dem Druckvolumenpuls. Anhand des Diagramms ist unmittelbar zu erkennen, dass aus dem Messsignal beispielsweise physiologische Parameter wie die Pulsamplitude, die Pulsbreite, die Blutmenge, die Pulsdauer, die Durchblutung, der Blutdruck, der Pulsdruck sowie die Herzfrequenz abgeleitet werden können. Zudem kann die Pulswellengeschwindigkeit ermittelt werden. Daraus können wiederum weitere verwandte physiologische Parameter abgeleitet werden. Beispielsweise ist bekannt, dass die Pulsamplitude mit der Körpertemperatur korreliert. Somit kann der Drucksensor der erfindungsgemäßen Messvorrichtung dazu genutzt werden, mit der Temperatur zusammenhängende physiologische Parameter zu bestimmen, wie die Körperoberflächentemperatur, die Körperdurchschnittstemperatur, die arterielle Bluttemperatur, die Körperkerntemperatur, die vom Metabolismus erzeugte Wärmemenge, die vom Körperinnerem zu den Kapillarenden fließende Wärmemenge sowie die Temperatur im Bereich der arteriell-venösen Kapillaren bzw. des diese umgebenden Gewebes.

Bei dem in Figur 2 dargestellten Ausführungsbeispiel ist die im Querschnitt dargestellte Sensoreinheit insgesamt mit der Bezugsziffer 1 bezeichnet. Die Sensoreinheit umfasst eine bioelektrische Impedanzmesseinheit mit einer Messelektrode 2. Die Messelektrode 2 ist als elektrisch leitendes Blech ausgebildet, deren in der Figur 2 oben dargestellte, horizontal verlaufende Oberfläche mit der Körperoberfläche des Benutzers in Kontakt gebracht wird, um dort elektrische Messsignale (Potentiale) zu erfassen. Die Messelektrode 2 ist beweglich, nämlich federnd gelagert, was in der Figur 2 schematisch angedeutet ist. Der Doppelpfeil in der Figur 2 illustriert die Beweglichkeit der Messelektrode 2. Unterhalb der Messelektrode 2 ist ein Drucksensor 3, zum Beispiel in Form eines piezoresistiven Elementes, angeordnet, der den von dem zu untersuchenden Körpergewebe über die Messeletrode 2 auf den Drucksensor 3 ausgeübten Druck detektiert. Dabei stützt sich der Drucksensor 3 an der Rückseite an einem fixen Teil 4 ab.

Die Messelektrode 2 und der Drucksensor 3 sind mit einer in den Figuren nicht näher dargestellten Auswertungseinheit der erfindungsgemäßen Messvorrichtung verbunden. Über diese Verbindung werden die Messsignale der bioelektrischen Impedanzmesseinheit und des Drucksensors 3 an die Auswertungseinheit übertragen. Die Auswertungsseinheit leitet aus den Messsignalen wenigstens einen physiologischen Parameter ab. Hierzu umfasst die Auswertungseinheit zum Beispiel einen geeignet programmierten Mikrocontroller mit Schnittstellen zur Digitalisierung der Messsignale und mit Schnittstellen zur Ausgabe der Resultate.

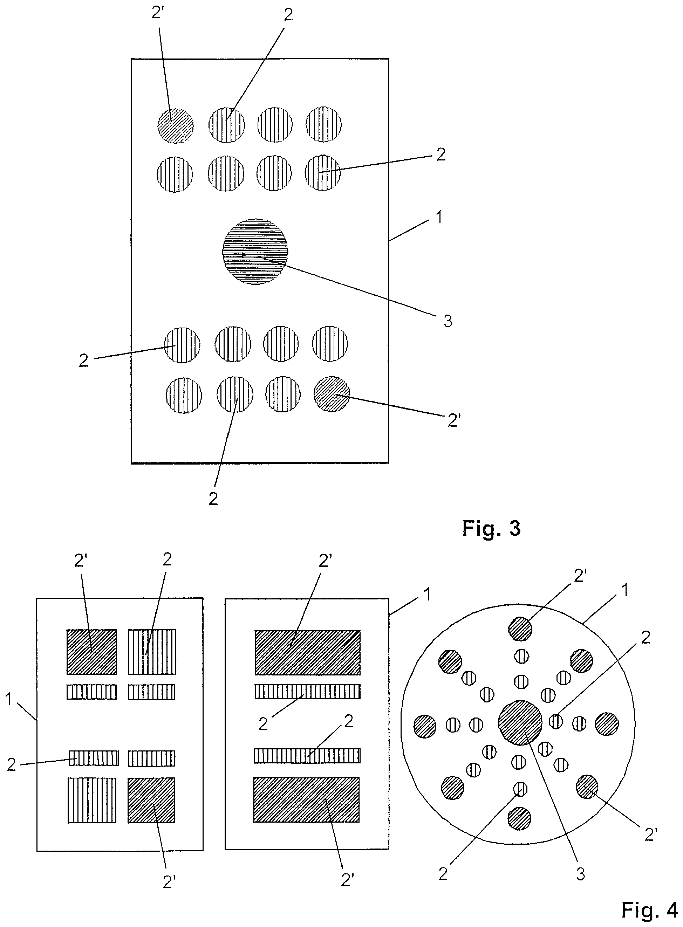

Die Figur 3 zeigt schematisch eine Draufsicht auf die bei einer Messung an der Körperoberfläche des Benutzers der Messvorrichtung anliegende Oberfläche der Sensoreinheit 1. Bei dem in Figur 3 dargestellten Ausführungsbeispiel ist der Drucksensor 3 zentral angeordnet. Um den Drucksensor 3 herum sind mehrere Messelektroden 2 matrixförmig angeordnet. Bei den Elektroden 2' handelt es sich um Einspeiseelektroden der bioelektrischen Impedanzmesseinheit. Über die Einspeiseelektroden 2' wird dem Körpergewebe ein Messstrom aufgeprägt. Die dadurch an der Körperoberfläche entstehenden Potentiale werden über die Messeletroden 2 erfasst. Die matrixförmige Anordnung der Mess- und Einspeiseelektroden 2 bzw. 2' erlaubt erfindungsgemäß eine Tiefenprofilanalyse von physiologischen Parametern. Je nach relativer Position der einspeisenden und messenden Elektroden 2 bzw. 2' ergibt sich ein unterschiedlicher Weg des elektrischen Stroms durch das Körpergewebe. Durch Vergleich der erfassten Potentiale kann eine tiefenaufgelöste Ableitung von physiologischen Parametern erfolgen, wie oben näher erläutert. Bei dem dargestellten Ausführungsbeispiel sind insgesamt 16 Elektroden 2 bzw. 2' vorgesehen. Eine wesentlich größere Zahl von Messelektroden kann sinnvoll sein, wenn zum Beispiel eine höhere Auflösung bei der Tiefenprofilanalyse gewünscht wird.

Die Figur 4 zeigt weitere Ausführungsbeispiele, wobei sich die Erfindung auf die linke Seite dieser Figur bezieht.. Bei den beiden linken Konfigurationen der Figur 4 ist wenigstens ein Drucksensor unter wenigstens einer der Elektroden 2, 2' angeordnet, wie in Figur 2 dargestellt. Bei der rechts in Figur 4 dargestellte Variante sind die Elektroden 2 und 2' in einer radialen Konfiguration um den zentralen Drucksensor 3 herum angeordnet. Sämtliche Konfigurationen eignen sich für eine tiefenaufgelöste Bestimmung von physiologischen Parametern, wie oben erläutert.

## Patentansprüche

1. Messvorrichtung zur nicht-invasiven Bestimmung von wenigstens einem physiologischen Parameter, mit einer diagnostischen Sensoreinheit (1) zur Erzeugung von Messsignalen und mit einer Auswertungseinheit zur Verarbeitung der Messsignale,
wobei die diagnostische Sensoreinheit (1) wenigstens einen Drucksensor (3) umfasst, der den von einem zu untersuchenden Körpergewebe lokal auf den Drucksensor (3) ausgeübten Druck detektiert, wobei die Auswertungseinheit zur Ableitung wenigstens eines physiologischen Parameters aus dem Messsignal des Drucksensors (3) eingerichtet ist,
wobei die Sensoreinheit (1) eine bioelektrische Impedanzmesseinheit umfasst, wobei die Auswertungseinheit zur Ableitung des wenigstens einen physiologischen Parameters zumindest aus den Messsignalen des Drucksensors (3) und der bioelektrischen Impedanzmesseinheit eingerichtet ist,
wobei der Drucksensor (3) mechanisch an eine Messelektrode (2) der bioelektrischen Impedanzmesseinheit gekoppelt ist und so den von dem zu untersuchenden Körpergewebe über die Messelektrode (2) lokal auf den Drucksensor (3) ausgeübten Druck detektiert,
wobei die Auswertungseinheit zur Ableitung des wenigstens einen physiologischen Parameters als Funktion der Tiefe im Körpergewebe eingerichtet ist,
**dadurch gekennzeichnet, dass**
die Messelektrode (2) an der diagnostischen Sensoreinheit (1) beweglich gelagert ist.

2. Messvorrichtung nach Anspruch 1, **dadurch gekennzeichnet, dass** der mindestens eine physiologische Parameter zeitabhängig erfasst wird.

3. Messvorrichtung nach Anspruch 1 bis 2, **dadurch gekennzeichnet, dass** die Auswertungseinheit zur Erstellung einer Tiefenquerschnittsprofilanalyse des wenigstens einen physiologischen Parameters eingerichtet ist.

4. Messvorrichtung nach Anspruch 1 bis 3, **dadurch gekennzeichnet, dass** die Stärke des zur Impedanzmessung aufgeprägten Stroms und/oder die Messfrequenz variiert wird.

5. Messvorrichtung nach Anspruch 1 bis 4, **dadurch gekennzeichnet, dass** die Einspeisselektroden (2') und/oder Messelektroden (2) symmetrisch angeordnet sind.

6. Messvorrichtung nach Anspruch 1 bis 5, **dadurch gekennzeichnet, dass** die Einspeisselektroden (2') und/oder Messelektroden (2) matrixförmig angeordnet sind.

7. Messvorrichtung nach Anspruch 1 bis 6, **dadurch gekennzeichnet, dass** die Einspeisselektroden (2') und/oder Messelektroden (2) kreissymmetrisch angeordnet sind.

8. Messvorrichtung nach Anspruch 8, **dadurch gekennzeichnet, dass** die Auswertungseinheit eingerichtet ist, aus dem zeitlichen Verlauf des Messsignals des Drucksensors (3) wenigstens einen der folgenden physiologischen Parameter abzuleiten: Druckvolumenpuls, Pulsamplitude, Pulsbreite, Pulsdauer, Durchblutung, Blutdruck, Pulsdruck, Herzfrequenz, Pulswellengeschwindigkeit, Körpertemperatur, metabolisch erzeugte Wärmemenge.

9. Messvorrichtung nach Anspruch 7 oder 8, **gekennzeichnet durch** Mittel zum Anpressen des Drucksensors (3) an die Oberfläche des Körpergewebes.

10. Messvorrichtung nach einem der Ansprüche 1 bis 9, **dadurch gekennzeichnet, dass** die Sensoreinheit (1) eine optische Messeinheit umfasst, die wenigstens eine Strahlungsquelle zum Bestrahlen des Körpergewebes und wenigstens einen Strahlungssensor zur Detektion der von dem Körpergewebe gestreuten und/oder transmittierten Strahlung umfasst, wobei die Auswertungseinheit zur Ableitung des wenigstens einen physiologischen Parameters zumindest aus den Messsignalen der bioelektrischen Impedanz-Messeinheit und der optischen Messeinheit eingerichtet ist.

11. Messvorrichtung nach einem der Ansprüche 1 bis 10, **dadurch gekennzeichnet, dass** die Sensoreinheit (1) einen Temperatursensor umfasst, wobei die Auswertungseinheit zur Ableitung des wenigstens einen physiologischen Parameters zumindest aus den Messsignalen der bioelektrischen Impedanz-Messeinheit und des Temperatursensors eingerichtet ist.

12. Messvorrichtung nach einem der Ansprüche 1 bis 11, **dadurch gekennzeichnet, dass** die Sensoreinheit (1) eine EKG-Einheit zur Erfassung eines EKG-Signals über zwei oder mehr EKG-Elektroden umfasst, wobei die Auswertungseinheit zur Ableitung des wenigstens einen physiologischen Parameters zumindest aus den Messsignalen der bioelektrischen Impedanz-Messeinheit und der EKG-Einheit eingerichtet ist.

13. Messvorrichtung nach einem der Ansprüche 1 bis 12, **dadurch gekennzeichnet, dass** der Drucksensor (3) mechanisch an eine Messelektrode (2) der EKG-Einheit und/oder der bioelektrischen Impedanzmesseinheit gekoppelt ist und so den von dem zu untersuchenden Körpergewebe über die Messelektrode (2) lokal auf den Drucksensor (3) ausgeübten Druck detektiert.

14. Messvorrichtung nach einem der Ansprüche 1 bis 13, **dadurch gekennzeichnet, dass** der Drucksensor (3) wenigstens ein piezoresistives Element umfasst.

15. Messvorrichtung nach einem der Ansprüche 1 bis 14, **dadurch gekennzeichnet, dass** die Auswertungseinheit zur Ableitung der Glukosekonzentration aus den Messsignalen eingerichtet ist.

16. Verfahren zur nicht-invasiven Bestimmung von wenigstens einem physiologischen Parameter mit einer Messvorrichtung nach einem der Ansprüche 1 bis 15, **dadurch gekennzeichnet, dass** der von einem zu untersuchenden Körpergewebe lokal auf einen Drucksensor (3) ausgeübte Druck detektiert wird, wobei der wenigstens eine physiologische Parameter aus dem detektierten Druck abgeleitet wird,
wobei mittels einer diagnostischen Sensoreinheit Messsignale erzeugt werden, aus denen der wenigstens eine physiologische Parameter als Funktion der Tiefe im Körpergewebe abgeleitet wird, wobei die Sensoreinheit umfasst:
wenigstens einen Drucksensor (3), der den von einem zu untersuchenden Körpergewebe lokal auf den Drucksensor (3) ausgeübten Druck detektiert, und/oder
eine optische Messeinheit, die wenigstens eine Strahlungsquelle zur Bestrahlung des Körpergewebes und wenigstens einen Strahlungssensor zur Detektion der von dem Körpergewebe gestreuten und/oder transmittierten Strahlung umfasst, und/oder
einen Temperatursensor und/oder
eine EKG-Einheit zur Erfassung eines EKG-Signals über zwei oder mehr EKG-Elektroden und/oder
eine bioelektrische Impedanz-Messeinheit.

## Claims

1. Measurement device for non-invasive determination of at least one physiological parameter, having a diagnostic sensor unit (1) for generating measurement signals, and having an evaluation unit for processing the measurement signals,
wherein the diagnostic sensor unit (1) comprises at least one pressure sensor (3) that detects the pressure exerted locally on the pressure sensor (3) by a body tissue to be examined, wherein the evaluation unit is set up for derivation of at least one physiological parameter from the measurement signal of the pressure sensor (3),
wherein the sensor unit (1) comprises a bioelectrical impedance measurement unit, wherein the evaluation unit is set up for derivation of the at least one physiological parameter at least from the measurement signals of the pressure sensor (3) and of the bioelectrical impedance measurement unit,
wherein that the pressure sensor (3) is mechanically coupled with a measurement electrode (2) of the bioelectrical impedance measurement unit, and thereby detects the pressure locally exerted on the pressure sensor (3) by the body tissue to be examined, by way of the measurement electrode (2),
wherein the evaluation unit is set up for derivation of the at least one physiological parameter as a function of the depth in the body tissue,
**characterized in that**
the measurement electrode (2) is movably mounted on the diagnostic sensor unit (1).

2. Measurement device according to claim 1, **characterized in that** the at least one physiological parameter is detected as a function of time.

3. Measurement device according to claim 1 or 2, characterized the evaluation unit is set up to produce a depth cross-sectional profile analysis of the at least one physiological parameter.

4. Measurement device according to one of claims 1 to 3, **characterized in that** the strength of the impressed current for impedance measurement and / or the measurement frequency is varied.

5. Measurement device according to one of claims 1 to 4, **characterized in that in that** the feed electrodes (2 ') and / or measurment electrodes (2) are arranged symmetrically.

6. Measurement device according to one of claims 1 to 5, **characterized in that** the feed electrodes (2 ') and / or measurment electrodes (2) are arranged in a matrix.

7. Measurement device according to one of claims 1 to 6, **characterized in that** the feed electrodes (2 ') and / or measurment electrodes (2) are arranged in a circular symmetry.

8. Measurement device according to claim 7, **characterized in that** the evaluation unit is set up for deriving at least one of the following physiological parameters from the time progression of the measurement signal of the pressure sensor (3): pressure volume pulse, pulse amplitude, pulse width, pulse duration, perfusion, blood pressure, pulse pressure, heart rate, pulse wave velocity, body temperature, metabolically generated heat amount.

9. Measurement device according 7 or 8, **characterized by** means for pressing the pressure sensor (3) against the surface of the body tissue

10. Measurement device according to one of claims 1 to 9, **characterized in that** the sensor unit (1) comprises an optical measurement unit that comprises at least one radiation source for irradiation of the body tissue and at least one radiation sensor for detection of the radiation scattered and/or transmitted by the body tissue, wherein the evaluation unit is set up for derivation of the at least one physiological parameter at least from the measurement signals of the bioelectrical impedance measurement unit and of the optical measurement unit.

11. Measurement device according to one of claims 1 to 10, **characterized in that** the sensor unit (1) comprises a temperature sensor, wherein the evaluation unit is set up for derivation of the at least one physiological parameter at least from the measurement signals of the pressure sensor and of the temperature sensor.

12. Measurement device according to one of claims 1 to 11, **characterized in that** the sensor unit (1) comprises an EKG unit for detection of an EKG signal by way of two or more EKG electrodes, wherein the evaluation unit is set up for derivation of the at least one physiological parameter at least from the measurement signals of the pressure sensor and of the EKG unit.

13. Measurement device according to one of claims 1 to 12, **characterized in that**, the pressure sensor (3) is mechanically coupled with a measurement electrode (2) of the EKG unit and/or of the bioelectrical impedance measurement unit, and thereby detects the pressure locally exerted on the pressure sensor (3) by the body tissue to be examined, by way of the measurement electrode (2).

14. Measurement device according to one of claims 1 to 13, **characterized in that** the pressure sensor (3) comprises at least one piezoresistive element.

15. Measurement device according to one of claims 1 to 14, **characterized in that** the evaluation unit is set up for derivation of the glucose concentration from the measurement signals.

16. Method according to claim 15, **characterized in that** measurement signals are generated by means of a diagnostic sensor unit, from which signals the at least one physiological parameter is derived as a function of the depth in the body tissue, wherein the sensor unit comprises:
at least one pressure sensor (3) that detects the pressure exerted locally on the pressure sensor (3) by a body tissue to be examined, and/or
an optical measurement unit that comprises at least one radiation source for irradiation of the body tissue and at least one radiation sensor for detection of the radiation scattered and/or transmitted by the body tissue, and/or
a temperature sensor, and/or
an EKG unit for detection of an EKG signal by way of two or more EKG electrodes, and/or
a bioelectrical impedance measurement unit.

## Revendications

1. Dispositif de mesure destiné à la détermination non invasive d'au moins un paramètre physiologique, comprenant une unité de détection (1) diagnostique servant à générer des signaux de mesure ainsi qu'une unité d'évaluation servant à traiter les signaux de mesure,
l'unité de capteur (1) diagnostique comprenant au moins un capteur de pression (3), qui détecte la pression exercée localement sur le capteur de pression (3) par un tissu corporel à examiner, l'unité d'évaluation étant configurée pour dériver au moins un paramètre physiologique du signal de mesure du capteur de pression (3),
l'unité de capteur (1) comprenant une unité de mesure de l'impédance bioélectrique, l'unité d'évaluation étant configurée pour dériver l'au moins un paramètre physiologique au moins en se basant sur les signaux de mesure du capteur de pression (3) et de l'unité de mesure de l'impédance bioélectrique, le capteur de pression (3) étant couplé mécaniquement à une électrode de mesure (2) de l'unité de mesure de l'impédance bioélectrique et détecte ainsi la pression exercée localement sur le capteur de pression (3) par le tissu corporel à examiner par le biais de l'électrode de mesure (2),
l'unité d'évaluation étant configurée pour dériver l'au moins un paramètre physiologique comme fonction de la profondeur dans le tissu corporel,
**caractérisé en ce que**
l'électrode de mesure (2) est montée mobile sur l'unité de capteur (1) diagnostique.

2. Dispositif de mesure selon la revendication 1, **caractérisé en ce que** l'au moins un paramètre physiologique est détecté en fonction du temps.

3. Dispositif de mesure selon les revendications 1 et 2, **caractérisé en ce que** l'unité d'évaluation est configurée pour établir une analyse de profil transversal en profondeur de l'au moins un paramètre physiologique.

4. Dispositif de mesure selon les revendications 1 à 3, **caractérisé en ce que** l'intensité du courant appliqué pour la mesure de l'impédance et/ou la fréquence de mesure sont variées.

5. Dispositif de mesure selon les revendications 1 à 4, **caractérisé en ce que** les électrodes d'alimentation (2') et/ou les électrodes de mesure (2) sont disposées de manière symétrique.

6. Dispositif de mesure selon les revendications 1 à 5, **caractérisé en ce que** les électrodes d'alimentation (2') et/ou les électrodes de mesure (2) sont disposées en forme de matrice.

7. Dispositif de mesure selon les revendications 1 à 6, **caractérisé en ce que** les électrodes d'alimentation (2') et/ou les électrodes de mesure (2) sont dans une disposition à symétrie circulaire.

8. Dispositif de mesure selon la revendication 8, **caractérisé en ce que** l'unité d'évaluation est configurée pour dériver de la variation dans le temps du signal de mesure du capteur de pression (3) au moins un des paramètres physiologiques suivants : le pouls, la pression puisée, la largeur de pulsation, la durée de pulsation, l'irrigation sanguine, la pression artérielle, la pression différentielle, la fréquence cardiaque, la vitesse d'onde de pouls, la température corporelle, la chaleur produite métaboliquement.

9. Dispositif de mesure selon la revendication 7 ou 8, **caractérisé par** des moyens servant à presser le capteur de pression (3) contre la surface du tissu corporel.

10. Dispositif de mesure selon l'une des revendications 1 à 9, **caractérisé en ce que** l'unité de capteur (1) comprend une unité de mesure optique, qui comprend au moins une source de rayonnement servant à irradier le tissu corporel et au moins un capteur de rayonnement servant à détecter le rayonnement diffusé et/ou transmis par le tissu corporel, l'unité d'évaluation étant configurée pour dériver l'au moins un paramètre physiologique au moins en se basant sur les signaux de mesure de l'unité de mesure de l'impédance bioélectrique et de l'unité de mesure optique.

11. Dispositif de mesure selon l'une des revendications 1 à 10, **caractérisé en ce que** l'unité de capteur (1) comprend un capteur de température, l'unité d'évaluation étant configurée pour dériver l'au moins un paramètre physiologique au moins en se basant sur les signaux de mesure de l'unité de mesure de l'impédance bioélectrique et du capteur de température.

12. Dispositif de mesure selon l'une des revendications 1 à 11, **caractérisé en ce que** l'unité de capteur (1) comprend une unité ECG servant à détecter un signal ECG par le biais de deux électrodes ECG ou plus, l'unité d'évaluation étant configurée pour dériver l'au moins un paramètre physiologique au moins en se basant sur les signaux de mesure de l'unité de mesure de l'impédance bioélectrique et de l'unité ECG.

13. Dispositif de mesure selon l'une des revendications 1 à 12, **caractérisé en ce que** le capteur de pression (3) est couplé mécaniquement à une électrode de mesure (2) de l'unité ECG et/ou de l'unité de mesure de l'impédance bioélectrique et détecte ainsi la pression exercée localement sur le capteur de pression (3) par le tissu corporel à examiner par le biais de l'électrode de mesure (2).

14. Dispositif de mesure selon l'une des revendications 1 à 13, **caractérisé en ce que** le capteur de pression (3) comprend au moins un élément piézorésistif.

15. Dispositif de mesure selon l'une des revendications 1 à 14, **caractérisé en ce que** l'unité d'évaluation est configurée pour dériver la concentration de glucose des signaux de mesure.

16. Procédé de détermination non invasive d'au moins un paramètre physiologique, comprenant un dispositif de mesure selon l'une des revendications 1 à 15, **caractérisé en ce que** la pression exercée localement sur un capteur de pression (3) par un tissu corporel à examiner est détectée, l'au moins un paramètre physiologique étant dérivé de la pression détectée,
des signaux de mesure étant générés au moyen d'une unité de détection diagnostique, l'au moins un paramètre physiologique étant dérivé desdits signaux comme fonction de la profondeur dans le tissu corporel, l'unité de détection comprenant :
au moins un capteur de pression (3), qui détecte la pression exercée localement sur le capteur de pression (3) par un tissu corporel à examiner et/ou une unité de mesure optique, qui comprend au moins une source de rayonnement servant à irradier le tissu corporel et au moins un capteur de rayonnement servant à détecter le rayonnement diffusé et/ou transmis par le tissu corporel, et/ou
un capteur de température et/ou
une unité ECG servant à détecter un signal ECG par le biais de deux électrodes ECG ou plus et/ou
une unité de mesure de l'impédance bioélectrique.
